(19) Europäisches Patentamt
European Patent Office
Office européen des brevets

(11) Veröffentlichungsnummer: **0 078 239**
**A2**

# (12) EUROPÄISCHE PATENTANMELDUNG

(21) Anmeldenummer: **82810437.2**

(22) Anmeldetag: **22.10.82**

(51) Int. Cl.[3]: **C 07 D 307/60**
C 07 D 401/04, C 07 D 471/04
//(C07D471/04, 235/00, 221/00)

(30) Priorität: **28.10.81 CH 6880/81**

(43) Veröffentlichungstag der Anmeldung:
**04.05.83 Patentblatt 83/18**

(84) Benannte Vertragsstaaten:
**BE CH DE FR GB IT LI NL SE**

(71) Anmelder: **CIBA-GEIGY AG**
**Patentabteilung Postfach**
**CH-4002 Basel(CH)**

(72) Erfinder: **Breitenstein, Werner, Dr.**
**St. Galler-Ring 179**
**CH-4054 Basel(CH)**

(72) Erfinder: **Baumann, Marcus, Dr.**
**Rührbergerstrasse 6**
**CH-4058 Basel(CH)**

(72) Erfinder: **Bosshard, Hans**
**Hohe Winde-Strasse 23**
**CH-4059 Basel(CH)**

(54) **Verfahren zur Herstellung von asymmetrisch substituierten Maleinsäureanhydriden und neue asymmetrisch substituierte Maleinsäureanhydride.**

(57) Verbindungen der Formel I

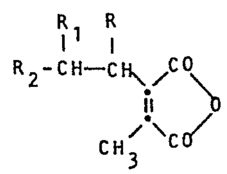

(I)

worin R, $R_1$ und $R_2$ die im Patentanspruch 1 angegebene Bedeutung haben, können nach einem neuen Verfahren z.B. dadurch hergestellt werden, dass man ein Salz des Imidazo [1,2-a]pyridin-2-(3H)-ons in Gegenwart einer Base mit Fumarsäure, Maleinsäure oder Maleinsäureanhydrid versetzt und in Gegenwart einer starken Säure zu einem Salz des 3-(1,2-Dicarboxyäthyl)-imidazo[1,2-a]pyridin-2-(3H)ons umsetzt, dieses mit einer Verbindung $CH_2=C(R_1)(R_2)$ umsetzt und das erhaltene Zwischenprodukt mit Eisessig und Natriumacetat behandelt. Die Verbindungen (I) können in an sich bekannter Weise in entsprechende Imide mit für Polymerisations- oder Polykondensationsreaktionen geeigneten funktionellen Gruppen übergeführt werden. Aus solchen Imiden lassen sich lichtvernetzbare Polymere herstellen. Aus den Anhydriden (I) können z.T. auch durch Umsetzung mit Salzen geeigneter Amine pharmazeutisch wirksame Benzofuranone und Homosalicylsäuren hergestellt werden.

EP 0 078 239 A2

0078239



CIBA-GEIGY AG
Basel (Schweiz)

6-13613/+

# Verfahren zur Herstellung von asymmetrisch substituierten Maleinsäureanhydriden und neue asymmetrisch substituierte Maleinsäureanhydride

Die Erfindung betrifft ein Verfahren zur Herstellung von asymmetrisch substituierten Maleinsäureanhydriden, die damit erhältlichen neuen asymmetrisch substituierten Maleinsäureanhydride sowie die zur Herstellung der asymmetrisch substituierten Maleinsäureanhydride entwickelten neuen Zwischenprodukte.

3-Methyl-4-(2-carboxyäthyl)-maleinsäureanhydrid (Hämatinsäureanhydrid) kann dadurch erhalten werden, dass man Aethyl-α-acetylglutarat oder γ-Oxo-δ-äthoxycarbonyl-capronsäureäthylester mit HCN, gegebenenfalls unter Zusatz katalytischer Mengen KCN, zu den entsprechenden Cyanhydrinen umsetzt und die Cyanhydrine zuerst mit konzentrierter HCl bei Temperaturen zwischen 90 und 100°C hydrolysiert und anschliessend auf Temperaturen zwischen 175 und 185°C erhitzt [vgl. z.B. J.Chem.Soc., 2621 (1928); Biochem.J., 45, 163 (1949) und Annalen der Chemie, 680, 60 (1964)]. Wegen der dabei erforderlichen drastischen Reaktionsbedingungen eignet sich dieses Verfahren nur zur Herstellung von Maleinsäureanhydriden mit hydrolysebeständigen Substituenten in 3- oder 4-Stellung. Zudem ist die Herstellung der dafür benötigten Ausgangsprodukte relativ kompliziert. Andererseits ist bekannt, dass man Dimethylmaleinsäureanhydrid durch Umsetzung bestimmter heterocyclischer, gegebenenfalls N-substituierter Amidine oder Amidinsalze mit Fumar- oder Maleinsäure bzw. Maleinsäureanhydrid und saure Hydrolyse der Reaktionsprodukte unter Rückflussbedingungen herstellen kann [vgl. z.B. DE-OS 22 33 889 und 22 33 862].

Es wurde nun ein neues, breit anwendbares und einfaches Verfahren zur Herstellung von asymmetrisch substituierten Maleinsäureanhydriden gefunden, nach welchem sich auch Maleinsäureanhydride mit hydrolyseempfindlichen Substituenten in 3- bzw. 4-Stellung, wie z.B. Nitril- oder Estergruppen aufweisenden Substituenten, herstellen lassen.

Gegenstand der Erfindung ist somit ein neues Verfahren zur Herstellung von Verbindungen der Formel I

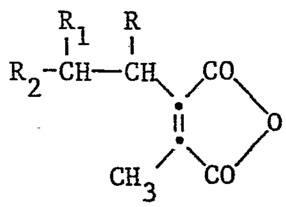

(I),

worin

R Wasserstoff, $C_{1-7}$-Alkyl, unsubstituiertes oder durch Halogen, Methoxy oder $C_{1-4}$-Alkyl substituiertes Phenyl,
$R_1$ Wasserstoff, $C_{1-7}$-Alkyl, Cyclopentyl, Cyclohexyl, unsubstituiertes oder durch Halogen, Methoxy oder $C_{1-4}$-Alkyl substituiertes Phenyl oder R und $R_1$ zusammen $-(CH_2)_3-$ oder $-(CH_2)_4-$ und
$R_2$ $-CO-C_{1-8}$-Alkyl, -COPhenyl, das durch $C_{1-4}$-Alkyl substituiert sein kann, -CN, $-COO-C_{1-4}$-Alkyl, -COO-Allyl oder -COOH bedeuten oder worin $R_1$ Wasserstoff ist und R und $R_2$ zusammen $-(CH_2)_3-CO-$ darstellen, wobei die Carbonylgruppe in dem so gebildeten Ring in m-Stellung zur Gruppe -CH- steht, indem man

a) bei R = H eine Verbindung der Formel II

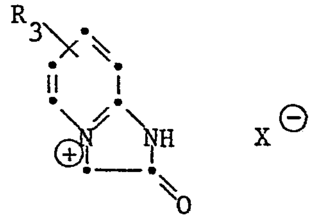

(II)

zuerst in Gegenwart einer Base mit Fumarsäure, Maleinsäure oder Maleinsäureanhydrid versetzt und anschliessend in Gegenwart einer starken Säure zu einer Verbindung der Formel III

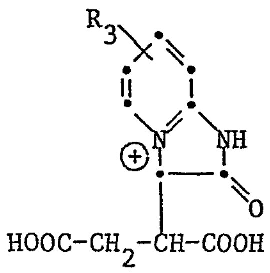 $X^{\ominus}$ (III)

umsetzt, die Verbindung der Formel III mit einer Verbindung der Formel IVa

$$CH_2=C(R_1)(R_2) \qquad (IVa)$$

zu einer Verbindung der Formel V

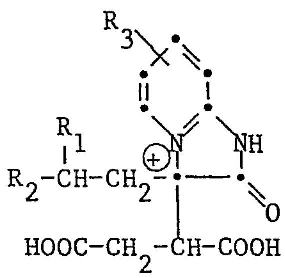 $X^{\ominus}$ (V)

umsetzt, die Verbindung der Formel V durch Erhitzen auf Temperaturen zwischen 80 und 160°C in ein Gemisch aus einer Verbindung der Formel I mit R = H und einer Verbindung der Formel VIa

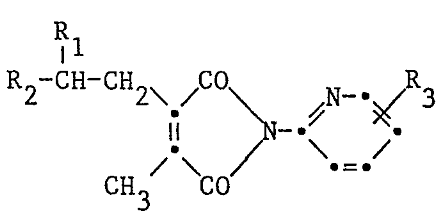 (VIa)

überführt und die Verbindung der Formel VIa mit wässriger Säure zu einer Verbindung der Formel I mit R = H hydrolysiert, oder

b) bei R ungleich H eine Verbindung der Formel II zuerst in Gegenwart einer Base mit einer Verbindung der Formel IVb

$$R'-CH=C(R_1)(R_2) \qquad (IVb)$$

versetzt und anschliessend bei Temperaturen zwischen 80 und 160°C mit Fumarsäure, Maleinsäure oder Maleinsäureanhydrid zu einem Gemisch aus einer Verbindung der Formel I mit R ungleich H und einer Verbindung der Formel VIb

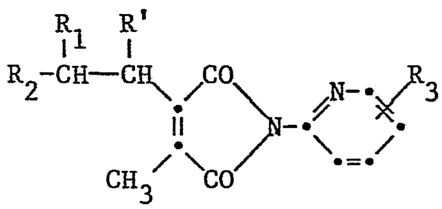

(VIb)

umsetzt und die Verbindung der Formel VIb mit wässriger Säure zu einer Verbindung der Formel I mit R ungleich H hydrolysiert. Dabei gilt für R, $R_1$ und $R_2$ das unter Formel I Angegebene, R' hat dieselbe Bedeutung wie R, ist aber ungleich H oder stellt bei $R_1$ = H zusammen mit $R_2$ $-(CH_2)_3-CO-$ dar, wobei die Carbonylgruppe in dem so gebildeten Ring in m-Stellung zur Gruppe -CH- steht, $R_3$ ist Wasserstoff oder Methyl und $X^{\ominus}$ bedeutet das Anion einer organischen oder anorganischen Säure.

Derartige Reaktionen mit Imidazopyridiniumsalzen waren bisher nicht bekannt. Insbesondere war es überraschend, dass beim Erhitzen der Verbindungen der Formel V bzw. der Umsetzung gemäss Verfahrensvariante b) mehr oder weniger gleichzeitig eine teilweise bis vollständige Eliminierung von 2-Aminopyridin bzw. methylsubstituiertem 2-Aminopyridin, eine Decarboxylierung und ein Ringschluss zum Anhydrid eintreten. Die Kombination dieser drei Reaktionen in einem Reaktionsschritt ist unerwartet und ungewöhnlich.

Die Verbindungen der Formel I sind neu, mit Ausnahme des Hämatinsäureanhydrids (R und $R_1$ = H und $R_2$ = -COOH). Die neuen Verbindungen der Formel I'

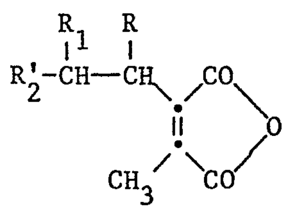

(I'),

worin für R und $R_1$ das unter Formel I Angegebene gilt und $R_2'$ dieselbe Bedeutung wie $R_2$ hat, aber bei R und $R_1$ = H ungleich -COOH ist, sind ebenfalls Gegenstand der Erfindung.

Alkylgruppen R, R' und $R_1$ sowie definitionsgemässe Alkylgruppen in Resten R, $R_1$ und $R_2$ oder $R_2'$ können geradkettig oder verzweigt sein. Als Beispiele solcher Gruppen seien genannt: die Methyl-, Aethyl-, n-Propyl-, Isopropyl-, n-Butyl-, sek- und tert-Butyl-, n-Pentyl-, 2-Pentyl-, n-Hexyl- und n-Heptylgruppe. Besonders bevorzugt sind Alkylgruppen mit 1-4 C-Atomen, ganz besonders Methyl und Aethyl. Sind Phenylgruppen R, R' oder $R_1$ durch Halogenatome substituiert, so handelt es sich z.B. um F, Cl oder Br. Phenylgruppen R, R' oder $R_1$ und Gruppen -COPhenyl sind vorzugsweise monosubstituiert. Besonders bevorzugt sind unsubstituiertes Phenyl und -COPhenyl.

$R_3$ ist vorzugsweise Wasserstoff.

$X^{\ominus}$ als Anion einer organischen Säure stellt z.B. das Anion der Trifluoressig-, Benzolsulfon-, p-Toluolsulfon- oder Methansulfonsäure dar. Als Anionen anorganischer Säuren kommen beispielsweise die Anionen von Halogenwasserstoffsäuren, der Salpeter-, Phosphor-, Schwefel- oder Fluorsulfonsäure in Betracht. Bevorzugt stellt $X^{\ominus}$ das Anion der Salpeter- oder Schwefelsäure oder einer Halogenwasserstoffsäure, besonders HCl oder HBr, dar.

Bevorzugt sind Verbindungen der Formel I, worin $R_1$ Wasserstoff ist und R und $R_2$ bzw. R und $R_2'$ zusammen $-(CH_2)_3-CO-$ darstellen oder worin R Wasserstoff, $C_{1-7}$-Alkyl oder Phenyl und $R_1$ Wasserstoff, $C_{1-7}$-Alkyl, Cyclopentyl, Cyclohexyl oder Phenyl darstellen, wobei bevorzugt mindestens eines von R und $R_1$ Wasserstoff ist, oder R und $R_1$ zusammen $-(CH_2)_3-$ oder $-(CH_2)_4-$ darstellen und $R_2$ bzw. $R_2'$ $-CO-C_{1-8}$-Alkyl, -COPhenyl, -CN, $-COOCH_3$, $-COOC_2H_5$ oder, wenn eines von R und $R_1$ ungleich Wasserstoff ist, -COOH, bedeuten, vor allem Verbindungen der Formel I, worin R und $R_1$ die oben angegebene Bedeutung haben und $R_2$ bzw. $R_2'$ $-CO-C_{1-8}$-Alkyl oder -COPhenyl darstellt, das durch $C_{1-4}$-Alkyl substituiert sein kann.

Besonders bevorzugt sind Verbindungen der Formel I, worin R und $R_1$ unabhängig voneinander Wasserstoff oder $C_{1-7}$-Alkyl, besonders $C_{1-4}$-Alkyl und vor allem Methyl, oder zusammen $-(CH_2)_3-$ oder $-(CH_2)_4-$ und $R_2$ bzw. $R_2'$ $-CO-C_{1-8}$-Alkyl, besonders $-CO-C_{1-5}$-Alkyl, darstellen. Ganz besonders bevorzugt sind Verbindungen der Formel I, worin eines von R und $R_1$ Wasserstoff und das andere Wasserstoff oder $C_{1-4}$-Alkyl, vor allem Methyl, und $R_2$ bzw. $R_2'$ $-CO-C_{1-5}$-Alkyl, besonders $-COCH_3$ oder $-COC_2H_5$, darstellen. Am meisten bevorzugt sind Verbindungen der Formel I, worin R Wasserstoff, $R_1$ $C_{1-4}$-Alkyl, besonders Methyl, und $R_2$ bzw. $R_2'$ $-COCH_3$ bedeuten.

Als spezifische Beispiele von neuen Verbindungen der Formel I seien genannt:

4-Methyl-3-(3-oxobutyl)-maleinsäureanhydrid,

4-Methyl-3-(3-oxopentyl)-maleinsäureanhydrid,

4-Methyl-3-(3-oxohexyl)-maleinsäureanhydrid,

4-Methyl-3-(3-phenyl-3-oxopropyl)-maleinsäureanhydrid,

4-Methyl-3-[3-(3- oder 4-methylphenyl)-3-oxopropyl]-maleinsäureanhydrid,

4-Methyl-3-[3-(4-äthylphenyl)-3-oxopropyl]-maleinsäureanhydrid,

4-Methyl-3-(2-methyl-3-oxobutyl)-maleinsäureanhydrid,

4-Methyl-3-(2-äthyl-3-oxobutyl)-maleinsäureanhydrid,

4-Methyl-3-(2-methyl-3-oxopentyl)-maleinsäureanhydrid,

4-Methyl-3-(2-cyano-1-methyläthyl)-maleinsäureanhydrid,
4-Methyl-3-(2-cyanoäthyl)-maleinsäureanhydrid,
4-Methyl-3-(2-cyano-1-phenyläthyl)-maleinsäureanhydrid,
4-Methyl-3-(1-methyl-3-oxobutyl)-maleinsäureanhydrid,
4-Methyl-3-(1-äthyl-3-oxobutyl)-maleinsäureanhydrid,
4-Methyl-3-(1-phenyl-3-oxobutyl)-maleinsäureanhydrid,
4-Methyl-3-[1-(4-chlorphenyl)-3-oxobutyl]-maleinsäureanhydrid,
4-Methyl-3-[1-(3- oder 4-methylphenyl)-3-oxobutyl]-maleinsäureanhydrid,
4-Methyl-3-[1-(4-methoxyphenyl)-3-oxobutyl]-maleinsäureanhydrid,
4-Methyl-3-(3-oxocyclohexyl)-maleinsäureanhydrid,
4-Methyl-3-(1,3-diphenyl-3-oxopropyl)-maleinsäureanhydrid,
4-Methyl-3-(2-methoxycarbonyl-1-methyläthyl)-maleinsäureanhydrid,
4-Methyl-3-(2-äthoxycarbonyläthyl)-maleinsäureanhydrid,
4-Methyl-3-(2-n-propoxycarbonyläthyl)-maleinsäureanhydrid,
4-Methyl-3-(2-carboxy-1-methyläthyl)-maleinsäureanhydrid,
4-Methyl-3-(2-carboxy-1-phenyläthyl)-maleinsäureanhydrid,
4-Methyl-3-(2-cyclohexyl-3-oxobutyl)-maleinsäureanhydrid,
4-Methyl-3-(2-phenyl-3-oxobutyl)-maleinsäureanhydrid,
4-Methyl-3-[2-(4-methoxyphenyl)-3-oxobutyl]-maleinsäureanhydrid.

Bei der Verfahrensvariante a) setzt man mit Vorteil Maleinsäure ein, während für die Verfahrensvariante b) Maleinsäureanhydrid bevorzugt ist.

Als Basen für die Umsetzung der Verbindungen der Formel II mit Fumarsäure, Maleinsäure oder Maleinsäureanhydrid bzw. mit den Verbindungen der Formel IVb können organische oder anorganische Verbindungen verwendet werden, wie tertiäre Amine, vor allem Trialkylamine oder cyclische Amine, z.B. Triäthylamin, Tri-n-butylamin, Pyridin, Picolin, Chinolin und Lutidin; Alkalimetall- oder Erdalkalimetallhydroxide, -carbonate oder -hydrogencarbonate oder Salze von Alkalimetallen oder Erdalkalimetallen mit schwachen Säuren, wie Carboxylate, vor allem Acetate. Bevorzugt verwendet man ein Alkalimetallhydroxid, vor allem NaOH oder KOH.

Die genannten Umsetzungen wie auch die weitere Umsetzung mit Verbindungen der Formel IVa gemäss Verfahrensvariante a) können in wässrigem, wässrig-organischem oder organischem Medium vorgenommen werden. Als organische Lösungsmittel eignen sich vor allem polare Lösungsmittel, wie Alkanole mit bis zu 6 C-Atomen, Aethylenglykolmonoalkyläther mit bis zu 4 C-Atomen im Alkylteil, N,N-Dialkylamide von aliphatischen Monocarbonsäuren mit 1-3 C-Atomen im Säureteil oder cyclische Amide, wie z.B. Methanol, Aethanol, n-Propanol, Isopropanol, Butanole und Hexanole, Aethylenglykolmonomethyl- und -monoäthyläther, N,N-Dimethylformamid, N,N-Dimethylacetamid, N,N-Dimethylpropionamid und N-Methylpyrrolidon.

Die Umsetzung der Verbindungen der Formel II mit Fumarsäure, Maleinsäure oder Maleinsäureanhydrid wird vorzugsweise in wässrigem Medium durchgeführt. Für die Umsetzungen mit Verbindungen der Formel IVa oder IVb wird ein wässrig-organisches, besonders ein wässrig-alkoholisches Medium, bevorzugt. Besonders bevorzugt sind für diese Reaktionen Gemische aus Wasser und Methanol.

Die Reaktionstemperaturen für die Umsetzung der Verbindungen der Formel II mit Fumarsäure, Maleinsäure oder Maleinsäureanhydrid bzw. den Verbindungen der Formel IVb liegen mit Vorteil zwischen 0 und 80°C, besonders 20 und 50°C. Für die Umsetzung der Verbindungen der Formel III mit den Verbindungen der Formel IVa werden Reaktionstemperaturen zwischen 0 und 50°C, besonders 20 und 40°C, bevorzugt.

Als starke Säuren können in der Verfahrensvariante a) sowohl organische als auch anorganische Verbindungen eingesetzt werden, z.B. Trifluoressigsäure, Benzolsulfonsäure, p-Toluolsulfonsäure, Methansulfonsäure, Halogenwasserstoffsäuren, Salpeter-, Phosphor-, Schwefel- oder Fluorsulfonsäure. Bevorzugt sind Salpetersäure, Schwefelsäure und Halogenwasserstoffsäuren, besonders HCl oder HBr.

Das Erhitzen der Verbindungen der Formel V und des nach Zusatz von Fumarsäure, Maleinsäure oder Maleinsäureanhydrid erhaltenen Reaktionsgemisches gemäss Verfahrensvariante b) kann mit oder ohne Zusatz eines hochsiedenden organischen Lösungsmittels und gegebenenfalls in Gegenwart eines Puffers, wie Natriumacetat, Kaliumpropionat, Natriumhydrogenphosphat oder Natriumcitrat, vorgenommen werden. Geeignete Lösungsmittel sind z.B. gegebenenfalls chlorierte aromatische Kohlenwasserstoffe, wie Toluol, Xylole, Chlorbenzol, N,N-Dimethylformamid, N,N-Dimethylacetamid, 1,2-Diäthoxyäthan, Propionsäure und Eisessig. Vorzugsweise wird die Ueberführung in Verbindungen der Formel I und Verbindungen der Formel VIa oder VIb bei Temperaturen zwischen 90 und 110°C und in Gegenwart eines hochsiedenden organischen Lösungsmittels, vor allem in Eisessig, vorgenommen.

Für die Hydrolyse der Verbindungen der Formel VIa oder VIb können wässrige organische oder wässrige anorganische Säuren, gegebenenfalls im Gemisch mit Verdünnungsmitteln, eingesetzt werden. Die Reaktionstemperaturen liegen mit Vorteil zwischen 20°C und dem Siedepunkt des Reaktionsmediums. Als Verdünnungsmittel eignen sich insbesondere mit Wasser mischbare organische Lösungsmittel, z.B. offenkettige oder cyclische Aether, wie 1,2-Dimethoxyäthan, Tetrahydrofuran, Tetrahydropyran und Dioxan. Bevorzugtes Verdünnungsmittel ist Tetrahydrofuran. Als organische Säuren kommen beispielsweise Trichloressigsäure, Trifluoressigsäure, Methansulfonsäure und p-Toluolsulfonsäure, als anorganische Säuren z.B. Schwefelsäure, Phosphorsäure, Salpetersäure, Salzsäure und Perchlorsäure in Betracht. Bevorzugt wird die Hydrolyse in wässrig anorganischer Säure, vor allem wässriger Schwefelsäure, im Gemisch mit einem mit Wasser mischbaren organischen Lösungsmittel, besonders Tetrahydrofuran, durchgeführt. Stellt $R_2$ in den Formeln VIa und VIb $-COO-C_{1-4}$-Alkyl oder -COO-Allyl dar, so wird auch diese Estergruppe hydrolysiert. Das bei der Hydrolyse abgespaltene 2-Aminopyridin oder methylsubstituierte 2-Aminopyridin kann praktisch quantitativ zurückgewonnen und wieder zur Herstellung der Ausgangsverbindungen der Formel II verwendet werden.

Die Ausgangsprodukte der Formeln II, IVa und IVb sind bekannt und können nach an sich bekannten Methoden hergestellt werden. Verbindungen der Formel II können z.B. nach dem in Ber., 57, 1381 und 2092 (1924) beschriebenen Verfahren erhalten werden.

Beim erfindungsgemässen Verfahren entstehen intermediär Verbindungen der Formel VII

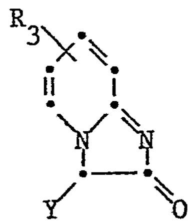

(VII),

worin $R_3$ die unter Formel II angegebene Bedeutung hat und Y HOOC-$CH_2$CH-COOH oder -CH(R)-CH($R_1$)($R_2$) darstellt. Die Verbindungen der Formel VII, die Zwischenprodukte der Formeln III und V und die daraus nach üblichen Methoden herstellbaren freien Basen sowie die Zwischenprodukte der Formeln VIa und VIb, die speziell für die Herstellung der erfindungsgemässen Verbindungen entwickelt wurden, sind neu und bilden ebenfalls einen Gegenstand der Erfindung. Dabei gilt in Bezug auf bevorzugte Bedeutungen von R, $R_1$, $R_2$, $R_3$ und $X^{\ominus}$ das oben Angegebene. Die genannten Zwischenprodukte können gewünschtenfalls in an sich bekannter Weise, z.B. durch Kristallisation, isoliert werden. Im allgemeinen wird jedoch auf eine solche Isolierung verzichtet. Gemische von Verbindungen der Formel I und VIa oder VIb können, falls erwünscht, auch in üblicher Weise getrennt werden, beispielsweise durch Destillation oder Chromatographie an Kieselgel.

Die Anhydride der Formel I können in an sich bekannter Weise in entsprechende Imide übergeführt werden, unter anderem in Imide mit funktionellen, für Polymerisations- oder Polykondensationsreaktionen geeigneten Gruppen der in der DE-OS 26 26 795 beschriebenen Art. Aus solchen Imiden mit funktionellen Gruppen lassen sich in ebenfalls an sich bekannter Weise lichtvernetzbare Polymere herstellen [vgl. z.B.

DE-OS 26 26 795], die beispielsweise zur Erzeugung von hochlichtempfindlichen Bildmaterialien, zur Herstellung von Druckplatten für das Offsetdruckverfahren, zur Herstellung von Photooffset-Lacken, für die unkonventionelle Photographie und vor allem als Photoresists zur Herstellung von gedruckten Schaltungen verwendet werden können.

Ausserdem stellen Verbindungen der Formel I, worin R und $R_1$ unabhängig voneinander Wasserstoff oder $C_{1-7}$-Alkyl oder zusammen $-(CH_2)_3-$ oder $-(CH_2)_4-$ und $R_2$ $-COCH_3$ bedeuten, wertvolle Ausgangsmaterialien zur Herstellung von durch eine cyclische oder N,N-disubstituierte Aminogruppe substituierten Benzofuranonen und Homosalicylsäuren oder Homosalicylsäurederivaten, Salzen und Isomeren davon dar, die Anwendung als Arzneimittelwirkstoffe finden. Diese Benzofuranone und Homosalicylsäuren können dadurch erhalten werden, dass man die genannten Verbindungen der Formel I mit Salzen geeigneter Amine umsetzt. Dabei entstehen Benzofuranone oder Gemische von Benzofuranonen und Homosalicylsäuren. Homosalicylsäuren können auch durch Hydrolyse der Benzofuranone hergestellt werden. Einige typische Vertreter sind in den Beispielen illustriert. Diese Benzofuranone und Homosalicylsäuren besitzen insbesondere eine ausgeprägte antiinflammatorische Wirkung, die sich z.B. durch Reduktion des Carrageeninpfotenoedems bei der Ratte ab einer Dosis von etwa 0,1 mg/kg p.o. analog der von Pasquale et al., Agents and Actions 5, 256 (1976) beschriebenen Methodik sowie im Adjuvans-Arthritis-Modell an der Ratte ab einer Dosis von etwa 10 mg/kg p.o. nachweisen lässt analog L. Riesterer und R. Jacques, Pharmacology 2, 288 (1969). Ausserdem hemmen die genannten Verbindungen in vitro ab einer Konzentration von etwa 10 µmol/l die Prostaglandinsynthese aus Arachidonsäure analog der von H.L. White und A.T. Glassman, Prostaglandin 7, 123 (1974), beschriebenen Methode.

Weiterhin weisen die genannten Benzofuranone und Homosalicylsäuren eine antinociceptive Wirkungskomponente auf, die sich z.B. aus der Reduktion des durch Phenyl-p-benzochinon induzierten Writhing-Syndroms

an der Maus ab einer Dosis von etwa 0,1 mg/kg p.o. ableiten lässt [Methodik: analog L.C. Hendershot und J. Forsaith, J. Pharmacol. exp. Therap. 125, 237 (1959)]. Ferner zeigen sie die Fähigkeit, aus dem Bereich des UV-Spektrums die auf der Epidermis Erytheme erzeugenden Strahlen (zwischen 290 und 320 nm) zu absorbieren, während die Substanzen für die bräunend wirkenden Strahlen von etwa 320 bis 400 nm durchlässig sind. Infolgedessen lassen sich diese Benzofuranone und Homosalicylsäuren als Antiinflammatorika, (periphere) Analgetika und/oder Lichtschutzmittel, z.B. für kosmetische Zwecke, verwenden.

Pharmazeutische Präparate, welche die genannten Benzofuranone oder Homosalicylsäuren oder Salze davon enthalten, eignen sich vor allem zur topischen Anwendung, ferner zur enteralen, wie oralen oder rektalen und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von der Warmblüterspezies, dem Alter und dem individuellen Zustand sowie von der Applikationsweise ab. Im Normalfall ist für einen etwa 75 kg schweren Warmblüter bei oraler Applikation eine ungefähre Tagesdosis von etwa 100 bis etwa 600 mg, vorteilhaft in mehreren gleichen Tagesdosen verteilt, zu veranschlagen. Solche pharmazeutische Präparate enthalten z.B. von etwa 10% bis etwa 80%, vorzugsweise von etwa 20% bis etwa 60% des Wirkstoffs. Pharmazeutische Präparate zu enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren unter Verwendung üblicher Träger- und/oder Hilfsstoffe, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfs-

stoffen, zu Tabletten oder Dragée-Kernen verarbeitet. Als topisch anwendbare pharmazeutische Präparate kommen in erster Linie Creme, Salben, Pasten, Schäume, Tinkturen und Lösungen in Frage, die etwa 0,1 bis etwa 5% des Wirkstoffs enthalten.

Beispiel 1:

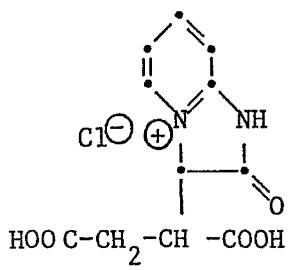

Ein Gemisch von 341 g (2 Mol) des Hydrochlorids von Imidazo-[1,2-a]pyridin-2-(3H)-on in 700 ml Wasser wird unter Rühren portionenweise mit einer Lösung von 80 g (2 Mol) Natronlauge in 200 ml Wasser versetzt. Anschliessend wird eine Lösung von 250,7 g (2,16 Mol) Maleinsäure in 600 ml Wasser so zugetropft, dass die Innentemperatur des Reaktionsgemisches zwischen 40°C und 45°C bleibt. Nach 30 Stunden bei Raumtemperatur (20-25°C) wird auf 5°C gekühlt, der gebildete Niederschlag abfiltriert, das Filtrat im Vakuum auf etwa die Hälfte eingeengt und das ausgefallene Produkt abgenutscht. Die vereinigten Rückstände werden mit wenig kaltem Methanol gewaschen und bei 50°C im Vakuum getrocknet. Es resultieren 400 g 3-(1,2-Dicarboxyäthyl)-imidazo[1,2-a]pyridin-2(3H)-on vom Smp. 193° (Zers). Das erhaltene Produkt wird bei Raumtemperatur während 6 Stunden mit 650 ml konz. Salzsäure verrührt. Nach Kühlen auf 5°C wird der Niederschlag abfiltriert, das Filtrat im Vakuum auf etwa die Hälfte eingeengt und das ausgefallene Produkt abgenutscht. Die vereinigten Rückstände werden mit Aceton gewaschen und bei 50°C im Vakuum getrocknet. Es resultieren 350 g (61% d.Th.) des Hydrochlorids von 3-(1,2-Dicarboxyäthyl)-imidazo[1,2-a]pyridin-2(3H)-on vom Smp. 205°C (Zers.). IR-Spektrum (KBr, $cm^{-1}$): u.a. 2970, 1780, 1735, 1660, 1535, 1170, 1125, 1080, 930, 770.

Beispiel 2:

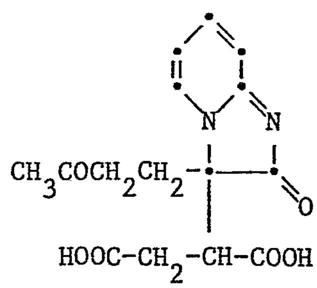

Ein Gemisch von 18,9 g (0,066 Mol) des Hydrochlorids von 3-(1,2-Dicarboxyäthyl)-imidazo[1,2-a]pyridin-2(3H)-on, 7 g (0,1 Mol) Methyl-vinylketon, 50 ml Methanol und 50 ml Wasser wird während 36 Stunden bei Raumtemperatur gerührt. Anschliessend wird das Produkt durch Zutropfen von ca. 15 ml 4N wässriger Natronlauge ausgefällt. Der Niederschlag wird genutscht, mit Wasser, Methanol und Aceton gewaschen und im Vakuum bei 50°C getrocknet. Es werden 8 g (38% d.Th.) 3-(1,2-Dicarboxyäthyl)-3-(3-oxobutyl)-imidazo[1,2-a]pyridin-2(3H)-on vom Smp. 195°C (Zers.) erhalten. $^{1}$H-NMR.-Spektrum (100 MHz, DMSO-$d_6$, $\delta$ in ppm): 1,80-2,60 (m,9H); 3,34 (m,1H); 6,90 (m,1H); 7,14 (m,1H); 7,86 (m,1H); 8,16 (m,1H). IR.-Spektrum (KBr, $cm^{-1}$): u.a. 1770, 1745, 1670, 1535, 1240, 1180.

Beispiel 3:

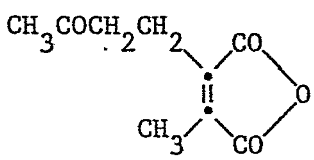

Ein Gemisch von 114,7 g (0,4 Mol) des Hydrochlorids von 3-(1,2-Dicarboxyäthyl)-imidazo[1,2-a]pyridin-2(3H)-on, 36,4 g (0,52 Mol) Methyl-vinylketon, 150 ml Methanol und 150 ml Wasser wird während 36 Stunden bei Raumtemperatur gerührt und anschliessend bei ca 45°C im Vakuum zur Trockene eingedampft.Das erhaltene Rohprodukt wird in 300 ml Eisessig aufgenommen, mit 15 g Natriumacetat versetzt und bis zur beendeten $CO_2$-Entwicklung am Rückfluss gekocht. Anschliessend wird das Lösungsmittel im Vakuum entfernt, der Rückstand mit einem

Gemisch von 150 ml 6M Schwefelsäure und 150 ml Tetrahydrofuran versetzt und während 8 Stunden bei 60°C gehalten. Nach dem Entfernen des Tetrahydrofurans im Vakuum wird das Reaktionsgemisch mit Wasser verdünnt, mit Methylenchlorid ausgezogen und über Kieselgel filtriert. Destillation des Rohproduktes im Hochvakuum (115°C-125°C/8 Pa) ergibt 34 g (47% d.Th.) 4-Methyl-3-(3-oxo-butyl)-maleinsäureanhydrid als spektroskopisch einheitliches blassgelbes Oel. Zur Elementaranalyse wird eine Probe mit Petroläther/Diäthyläther an Kieselgel chromatographiert. $^1$H-NMR.-Spektrum (100 MHz, $CDCl_3$, $\delta$ in ppm): 2,16 (s,3H); 2,19 (s,3H); 2,60-3,00 (m,4H). IR.-Spektrum ($CH_2Cl_2$, $cm^{-1}$): u.a. 1770, 1725, 1170, 1125, 925.

Elementaranalyse für $C_9H_{10}O_4$ (182,2):

Ber.: C 59,3% H 5,5%

Gef.: C 59,4% H 5,6%.

Beispiel 4: 4-Methyl-3-(3-oxopentyl)-maleinsäureanhydrid wird auf analoge Weise wie in Beispiel 3 beschrieben hergestellt, unter Verwendung von Aethyl-vinylketon anstelle von Methyl-vinylketon. Man erhält ein blassgelbes Oel. $^1$H-NMR.-Spektrum (100 MHz, $CDCl_3$, $\delta$ in ppm): 1,06 (t,J = 7 Hz, 3H); 2,13 (s,3H); 2,44 (q, J=7 Hz, 2H); 2,58-2,92 (m,4H). IR.-Spektrum ($CH_2Cl_2$, $cm^{-1}$): u.a. 1780, 1735, 1120, 935.

Elementaranalyse für $C_{10}H_{12}O_4$ (196,2):

Ber.: C 61,2% H 6,2%

Gef.: C 61,1% H 6,4%.

Beispiel 5: 4-Methyl-3-(3-phenyl-3-oxopropyl)-maleinsäureanhydrid wird auf analoge Weise wie in Beispiel 3 beschrieben hergestellt unter Verwendung von Phenyl-vinylketon anstelle von Methyl-vinylketon; Smp. 105°C-107°C. $^1$H-NMR.-Spektrum (100 MHz, $CDCl_3$, $\delta$ in ppm): 2,22 (s,3H); 2,80-3,00 (m,2H); 3,32-3,50 (m,2H); 7,38-7,70 (m,3H); 7,90-8,03 (m,2H). IR.-Spektrum (KBr, $cm^{-1}$): u.a. 1790, 1710, 1280, 1220, 1135, 980, 930, 895, 750, 735. Elementaranalyse für $C_{14}H_{12}O_4$ (244,2): Ber.: C 68,8% H 5,0%, Gef.: C 68,8% H 5,1%.

Beispiel 6: 4-Methyl-3-(2-methyl-3-oxobutyl)-maleinsäureanhydrid wird auf analoge Weise wie in Beispiel 3 beschrieben hergestellt unter Verwendung von 3-Methyl-3-buten-2-on anstelle von Methyl-vinylketon. Man erhält ein blassgelbes Oel. $^1$H-NMR.-Spektrum (100 MHz, $CDCl_3$, $\delta$ in ppm): 1,24 (d, J = 7 Hz, 3H); 2,12 (s,3H); 2,20 (s,3H); 2,40 (m,1H); 2,71-3,26 (m,2H). IR.-Spektrum (flüssig, $cm^{-1}$): u.a. 1780, 1720, 1285, 1180, 1120, 730. Elementaranalyse für $C_{10}H_{12}O_4$ (196,2): Ber.: C 61,2% H 6,2% O 32,6%, Gef.: C 61,0% H 6,3% O 32,5%.

Beispiel 7: 4-Methyl-3-(2-methyl-3-phenyl-3-oxopropyl)-maleinsäureanhydrid wird auf analoge Weise wie in Beispiel 3 beschrieben hergestellt, unter Verwendung von Phenyl-isopropenylketon anstelle von Methylvinylketon. Man erhält farblose Kristalle, die aus Isopropyläther umkristallisiert bei 89-90°C schmelzen. $^1$H-NMR-Spektrum (250 MHz, $CDCl_3$, $\delta$ in ppm): 1,30 (d, J= 7 Hz, 3H); 2,16 (s, 3H); 2,6 (m, 1H); 3,02 (m, 1H); 4,02 (m, 1H); 7,46-7,68 (m, 3H); 7,96 (m, 2H). Elementaranalyse für $C_{15}H_{14}O_4$(258,3): Ber. C 69,8% H 5,5% O 24,8%, Gef.: C 69,8% H 5,6% O 24,9%.

Beispiel 8:

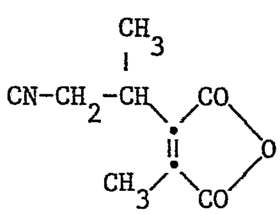

Zu einem Gemisch von 17 g (0,1 Mol) des Hydrochlorids von Imidazo-[1,2-a]pyridin-2(3H)-on in 40 ml Wasser werden unmittelbar nacheinander Lösungen von 4,4 g (0,11 Mol) Natronlauge in 30 ml Wasser und von 8,7 g (0,13 Mol) Crotonsäurenitril in 70 ml Methanol gegeben. Nach dem Rühren bei Raumtemperatur während 36 Stunden wird bei ca. 45°C im Vakuum zur Trockene eingedampft, der Rückstand in 120 ml Eisessig aufgenommen und nach Zugabe von 10,8 g (0,11 Mol) Maleinsäureanhydrid und 4 g Natriumacetat bis zur beendeten $CO_2$-Entwicklung am Rückfluss gekocht. Das Lösungsmittel wird im Vakuum abdestilliert, das Rohprodukt in einem Gemisch von 60 ml 6M Schwefelsäure und 60 ml Tetrahydrofuran aufgenommen und während 8 Stunden bei 60°C gehalten. Nach dem Entfernen des Tetrahydrofurans im Vakuum wird mit Wasser verdünnt und mit Methylenchlorid extrahiert. Die organische Phase wird getrocknet, eingedampft und der Rückstand mit Petroläther/ Methylenchlorid an 500 g Kieselgel chromatographiert. Destillation der reinen Fraktionen im Kugelrohr (140°C/10 Pa) ergibt 7,1 g (40% d.Th.) 3-(2-Cyano-1-methyläthyl)-4-methyl-maleinsäureanhydrid als blassgelbes Oel. (Kristallisiert nach längerem Stehenlassen; Smp. 37-39°C). $^1$H-NMR-Spektrum (100 MHz, $CDCl_3$, $\delta$ in ppm): 1,48 (d, J = 7 Hz, 3H); 2,21 (s,3H); 2,70-2,84 (m,2H); 3,28 (m,1H). IR.-Spektrum ($CH_2Cl_2$, $cm^{-1}$): u.a. 1790, 1275, 1150, 930, 905.

Elementaranalyse für $C_9H_9NO_3$ (179,2):

Ber.: C 60,3% H 5,1% N 7,8%
Gef.: C 60,5% H 5,2% N 7,8%.

<u>Beispiel 9:</u> 4-Methyl-3-(1-methyl-3-oxobutyl)-maleinsäureanhydrid wird auf analoge Weise wie in Beispiel 8 beschrieben hergestellt unter Verwendung von 3-Penten-2-on anstelle von Crotonsäurenitril. Man erhält ein blassgelbes Oel. $^1$H-NMR.-Spektrum (100 MHz, $CDCl_3$, $\delta$ in ppm): 1,26 (d, J= 7 Hz, 3H); 2,12 (s,6H); 2,60-3,50 (m,3H). IR-Spektrum (flüssig, $cm^{-1}$): u.a. 1780, 1740, 1370, 1280, 1180, 935, 910, 740.

Elementaranalyse für $C_{10}H_{12}O_4$ (196,2):

Ber.: C 61,2% H 6,2%
Gef.: C 61,1% H 6,2%.

Beispiel 10: 4-Methyl-3-(3-oxocyclohexyl)-maleinsäureanhydrid wird auf analoge Weise wie in Beispiel 8 beschrieben hergestellt unter Verwendung von Cyclohex-2-en-1-on anstelle von Crotonsäurenitril. Smp. 106°C-108°C. $^1$H-NMR.-Spektrum (100 MHz, $CDCl_3$, $\delta$ in ppm): 1,60-2,57 (m,7H); 2,13 (s,3H); 2,71-3,27 (m,2H). IR.-Spektrum ($CHCl_3$, $cm^{-1}$): u.a. 1790, 1730, 1290, 1270, 1135, 940, 915, 735.

Elementaranalyse für $C_{11}H_{12}O_4$ (208,2):

Ber.: C 63,5% H 5,8%
Gef.: C 63,5% H 5,7%.

Beispiel 11: 3-(1,3-Diphenyl-3-oxopropyl)-4-methyl-maleinsäureanhydrid wird auf analoge Weise wie in Beispiel 8 beschrieben hergestellt unter Verwendung von 1,3-Diphenyl-2-propenon anstelle von Crotonsäurenitril. $^1$H-NMR.-Spektrum (100 MHz, $CDCl_3$, $\delta$ in ppm): 2,19 (s,3H); 3,49-4,72 (ABX-System, 3H); 7,23-7,60 (m,8H); 7,89-8,02 (m,2H). IR.-Spektrum ($CH_2Cl_2$, $cm^{-1}$): u.a. 1790, 1720, 1620, 1510, 1460, 1220, 990, 935, 910.

Elementaranalyse für $C_{20}H_{16}O_4$ (320,3):

Ber.: C 75,0% H 5,0%
Gef.: C 75,1% H 5,1%.

Beispiel 12:

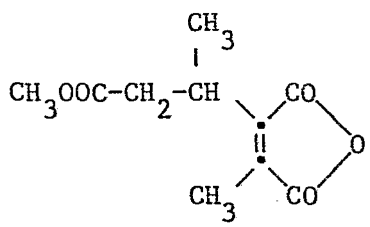

+

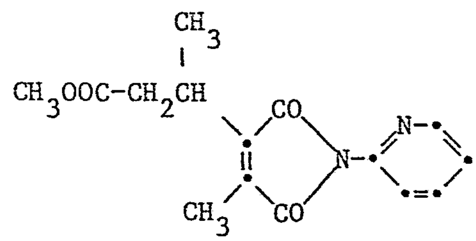

Zu einem Gemisch von 17 g (0,1 Mol) des Hydrochlorids von Imidazo-[1,2-a]pyridin-2(3H)-on in 40 ml Wasser werden nacheinander Lösungen von 4 g (0,1 Mol) Natronlauge in 30 ml Wasser und von 14 g (0,14 Mol) Crotonsäuremethylester in 70 ml Methanol gegeben. Nach dem Rühren bei Raumtemperatur während 72 Stunden wird bei ca. 45°C im Vakuum zur Trockene eingedampft, das Rohprodukt in 150 ml Eisessig aufgenommen und nach Zugabe von 10,8 g (0,11 Mol) Maleinsäureanhydrid und 5 g Natriumacetat bis zur beendeten $CO_2$- Entwicklung am Rückfluss gekocht. Das Lösungsmittel wird im Vakuum entfernt und der Rückstand zwischen Wasser und Methylenchlorid verteilt. Das nach dem Trocknen und Eindampfen der organischen Phase erhaltene Rohprodukt wird mit Petroläther/Diäthyläther an 600 g Kieselgel chromatographiert. Es werden 6,4 g (30% d.Th.) 3-(2-Methoxycarbonyl-1-methyläthyl)-4-methyl-maleinsäureanhydrid und 7,2 g (25% d.Th.) 3-(2-Methoxycarbonyl-1-methyläthyl)-4-methyl-N-(2-pyridyl)-maleinsäureimid erhalten.

Charakterisierung des 3-(2-Methoxycarbonyl-1-methyläthyl)-4-methylmaleinsäureanhydrids: $^1$H-NMR.-Spektrum (100 MHz, $CDCl_3$, $\delta$ in ppm): 1,34 (d, J = 7 Hz, 3H); 2,13 (s,3H); 2,50-3,04 (m, 2H); 3,32 (m,1H); 3,65 (s,3H). IR.-Spektrum ($CH_2Cl_2$, $cm^{-1}$): u.a. 1790, 1760, 1450, 1280, 1210, 1185, 910.

Elementaranalyse für $C_{10}H_{12}O_5$ (212,2):

Ber.: C 56,6% H 5,7%
Gef.: C 56,7% H 5,7%.

Charakterisierung des 3-(2-Methoxycarbonyl-1-methyläthyl)-4-methyl-N-(2-pyridyl)-maleinsäureimids: $^1$H-NMR.-Spektrum (100 MHz, $CDCl_3$, $\delta$ in ppm): 1,37 (d, J = 7 Hz, 3H); 2,11 (s,3H); 2,53-3,08 (m,2H); 3,38 (m,1H); 3,65 (s,3H); 7,19-7,39 (m,2H); 7,81 (m,1H); 8,61 (m,1H). IR.-Spektrum ($CH_2Cl_2$, $cm^{-1}$): u.a. 1740, 1610, 1485, 1450, 1395, 1185.

Elementaranalyse für $C_{15}H_{16}N_2O_4$ (288,3):

Ber.: C 62,5% H 5,6% N 9,7%.
Gef.: C 62,4% H 5,7% N 9,9%.

Beispiel 13: Zu einem Gemisch von 51,1 g (0,3 Mol) des Hydrochlorids von Imidazo[1,2-a]pyridin-2(3H)-on in 100 ml Wasser und 100 ml Methanol werden unter Rühren nacheinander 12 g (0,3 Mol) Natronlauge in 150 ml Methanol und 150 ml Wasser sowie 33,6 g (0,39 Mol) Crotonsäure gegeben. Nach Rühren bei Raumtemperatur während 72 Stunden wird bei ca. 45°C im Vakuum zur Trockene eingedampft, das Rohprodukt in 500 ml Eisessig aufgenommen und nach Zugabe von 5 g Natriumacetat und 32,4 g (0,33 Mol) Maleinsäureanhydrid bis zur beendeten $CO_2$-Entwicklung am Rückfluss gekocht. Das Lösungsmittel wird im Vakuum entfernt, das Rohprodukt in einem Gemisch von 200 ml 6 M Schwefelsäure und 200 ml Tetrahydrofuran aufgenommen und während 8 Stunden bei 60°C gehalten. Nach dem Entfernen von Tetrahydrofuran im Vakuum wird mit Wasser verdünnt und mit Methylenchlorid extrahiert. Die organische Phase wird getrocknet, eingedampft und der Rückstand mit Methylenchlorid/Diäthyläther über Kieselgel filtriert. Es werden 15 g (25% d.Th.) 3-(2-Carboxy-1-methyläthyl)-4-methylmaleinsäureanhydrid als chromatographisch einheitlicher Festkörper vom Smp. 86°C-88°C erhalten. Umkristallisation aus Diäthyläther/Petroläther ergibt farblose Kristalle vom Smp. 90°C-92°C. $^1$H-NMR.-Spektrum (100 MHz, $CDCl_3$, $\delta$ in ppm): 1,37 (d,J = 7 Hz, 3H); 2,12 (s,3H); 2,57-3,50 (m,3H); 10,9 (br.s, COOH). IR.-Spektrum (KBr, $cm^{-1}$): u.a. 1790, 1735, 1440, 1415, 1280, 1240, 1160, 960, 910, 740.

Elementaranalyse für $C_9H_{10}O_5$ (198,2):

Ber.: C 54,5% H 5,1%.
Gef.: C 54,5% H 5,3%.

Beispiel 14: Ein Gemisch aus 86 g (0,3 Mol) Hydrochlorid von 3-(1,2-Dicarboxyäthyl)-imidazo[1,2-a]pyridin-2(3H)-on, 41,2 g (0,42 Mol) 3-Aethyl-3-buten-2-on, 110 ml Methanol und 110 ml Wasser wird während 36 Stunden bei Raumtemperatur gerührt und anschliessend bei ca. 45°C im Vakuum zur Trockene eingedampft. Das erhaltene Rohprodukt wird in 225 ml Eisessig aufgenommen, mit 11 g Natriumacetat versetzt und bis zur beendeten $CO_2$-Entwicklung am Rückfluss gekocht. Anschliessend wird das Lösungsmittel im Vakuum entfernt, der Rückstand mit einem Gemisch aus 110 ml 6 M Schwefelsäure und 110 ml Tetrahydrofuran versetzt und während 8 Stunden am Rückfluss erhitzt. Nach dem Entfernen von Tetrahydrofuran im Vakuum wird das Reaktionsgemisch mit Wasser verdünnt und mit Methylenchlorid extrahiert. Das nach dem Trocknen und Eindampfen der organischen Phase verbleibende Rohprodukt wird mit Methylenchlorid an Kieselgel chromatographiert. Die anschliessende Destillation (110-120°C/13 Pa) ergibt 3-(2-Aethyl-3-oxobutyl)-4-methylmaleinsäureanhydrid als blassgelbes Oel.

Beispiel 15: Zu einem Gemisch aus 51,2 g ( 0,3 Mol ) Hydrochlorid von Imidazo[1,2-a]pyridin-2(3H)-on in 120 ml Wasser werden unmittelbar nacheinander Lösungen von 13,2 g (0,33 Mol) Natronlauge in 90 ml Wasser und 48,4 g (0,39 Mol) 1-Acetylcyclohexen in 210 ml Methanol gegeben. Nach Rühren bei Raumtemperatur während 24 Stunden wird bei ca. 45°C im Vakuum zur Trockene eingedampft, der Rückstand in 240 ml Eisessig aufgenommen und nach Zugabe von 29,4 g (0,3 Mol) Maleinsäureanhydrid und 7,5 g Natriumacetat bis zur beendeten $CO_2$-Entwicklung am Rückfluss gekocht. Das Lösungsmittel wird im Vakuum abdestilliert, das Rohprodukt in einem Gemisch aus 180 ml 6 M Schwefelsäure und 180 ml Tetrahydrofuran aufgenommen und während 8 Stunden am Rückfluss erhitzt. Nach dem Entfernen von Tetrahydrofuran im Vakuum wird mit Wasser verdünnt und mit Methylenchlorid extrahiert. Die organische Phase wird getrocknet, eingedampft und der Rückstand mit Petroläther/Methylenchlorid an Kieselgel chromatographiert. Die anschliessende Destillation (110-115°C/13 Pa) ergibt 4-Methyl-3-(1,2-tetramethylen-3-oxobutyl)maleinsäureanhydrid als blassgelbes Oel.

Beispiel 16: Ein Gemisch aus 14,9 g (0,082 Mol) 4-Methyl-3-(3-oxobutyl)-maleinsäureanhydrid und 5 g (0,082 Mol) Aethanolamin wird während 6 Stunden im offenen Kolben bei 120°C gehalten. Die anschliessende Destillation im Kugelrohr (150°C/6 Pa) ergibt 9,4 g (50% d.Th.) N-(2-Hydroxyäthyl)-4-methyl-3-(3-oxobutyl)-maleinsäureimid als gelbes Oel. $^1H$-NMR-Spektrum (100 MHz, $CDCl_3$, $\delta$ in ppm): 1,95 (s,3H); 2,10 (s,3H); 2,45-2,85 (m,4H); 3,12 (br.s, 1H , austauschbar mit $D_2O$); 3,4-3,75 (m,4H). IR-Spektrum ($CH_2Cl_2$, $cm^{-1}$): u.a. 1710, 1400, 1360, 1165, 1025.

Elementaranalyse für $C_{11}H_{15}NO_4$ (225,2):

| | | | |
|---|---|---|---|
| Ber.: | C 58,7% | H 6,7% | N 6,2% |
| Gef.: | C 58,5% | H 6,9% | N 6,4%. |

Zu einer Lösung von 5 g Copolymer aus Methylvinyläther und Maleinsäureanhydrid in 86 ml trockenem Tetrahydrofuran und 0,1 ml Pyridin werden 0,01598 Mol N-(2-Hydroxyäthyl)-4-methyl-3-(3-oxobutyl)-maleinsäureimid beigefügt. Die Lösung wird 48 Stunden bei 66°C gerührt. 20 ml Lösung werden aus Diäthyläther ausgefällt und getrocknet. Aus der Elementaranalyse errechnet sich eine Aufnahme des N-(2-Hydroxyäthyl)-4-methyl-3-(3-oxobutyl)-maleinsäureimids von 51,8% der eingesetzten Menge.

Mit der obigen Lösung werden gebürstete Aluminiumplatten beschichtet. Die Platten werden durch eine Negativvorlage (Stufenkeil Stouffer 21-Step-Sensitivity Guide) aus einem Abstand von 70 cm mit einer 400 Watt Metallhalogenidlampe bestrahlt. Dann wird die Platte in Tetrahydrofuran und Natriumbicarbonatlösung entwickelt, bis das Negativbild erscheint.

Letzte abgebildete Stufe nach 1 Minute Belichtung: Stufe 1
Letzte abgebildete Stufe nach 3 Minuten Belichtung: Stufe 2
Letzte abgebildete Stufe nach 6 Minuten Belichtung: Stufe 4.

Beispiel 17: Ein Gemisch von 18,2 g (0,1 Mol) 4-Methyl-3-(3-oxobutyl)-maleinsäureanhydrid und 22 g (0,105 Mol) Morpholiniumbenzoat in 400 ml Benzol wird während 48 Stunden am Wasserabscheider unter Rückfluss erhitzt. Das Benzol wird im Vakuum entfernt, der Rückstand in Methylenchlorid aufgenommen und die organische Phase zweimal mit gesättigter Natriumbicarbonatlösung extrahiert. Das nach dem Trocknen und Entfernen des Methylenchlorids verbleibende Rohprodukt wird mit Petroläther/Diäthyläther an Kieselgel chromatographiert. Es werden blassgelbe Kristalle erhalten, die aus Methylenchlorid/Diäthyläther umkristallisiert werden. Man erhält so das 3-Methyl-6-morpholino-2(3H)-benzofuranon vom Smp. 118-121°C.

Beispiel 18: Ein Gemisch aus 15,3 g (0,065 Mol) 4-Methyl-3-(1,2-tetramethylen-3-oxobutyl)-maleinsäureanhydrid und 13,5 g (0,07 Mol) Pyrrolidiniumbenzoat in 400 ml Benzol wird während 60 Stunden am Wasserabscheider unter Rückfluss erhitzt. Das Benzol wird im Vakuum entfernt und der Rückstand zwischen Methylenchlorid und gesättigter Natriumbicarbonatlösung verteilt. Das nach dem Trocknen und Entfernen des Methylenchlorids verbleibende Rohprodukt wird mit Petroläther/Diäthyläther an Kieselgel chromatographiert. Anschliessende Umkristallisation aus Diäthyläther/Petroläther ergibt 3-Methyl-6-pyrrolidin-1-yl-4,5-tetramethylen-benzofuran-2(3H)-on vom Smp. 99-101°C.

Beispiel 19: Zu einem Gemisch aus 14,7 g (0,063 Mol) 3-Methyl-6-morpholino-2(3H)-benzofuranon in 100 ml Chloroform wird bei 0-5°C unter Rühren eine kalte Lösung von Chlor in Chloroform getropft, bis im Dünnschichtchromatogramm kein Edukt mehr sichtbar ist. Das Reaktionsgemisch wird mit Methylenchlorid verdünnt und nacheinander mit 10%iger Natriumthiosulfatlösung, verdünnter Natriumbicarbonatlösung und Wasser

gewaschen. Das nach dem Trocknen und Eindampfen der organischen Phase verbleibende Rohprodukt wird mit Petroläther/Diäthyläther an Kieselgel chromatographiert. Nach Umkristallisation der reinen Fraktionen aus Diäthyläther/Petroläther erhält man das 5-Chlor-3-methyl-6-morpholino-2-(3H)-benzofuranon vom Smp. 103-105°C.

Beispiel 20: 5,4 g (0,02 Mol) 5-Chlor-3-methyl-6-morpholino-benzofuran-2(3H)-on werden in 40 ml 1n Natronlauge bei 50°C gelöst. Nach dem Abkühlen wird mit Diäthyläther gewaschen und die wässrige Phase anschliessend mit 1n Salzsäure auf pH 2,0 gestellt. Das dabei gebildete Oel wird in Diäthyläther aufgenommen.

Nach dem Verdampfen des Diäthyläthers wird 2-(5-Chlor-2-hydroxy-4-morpholinophenyl)-propionsäure als farblose Kristalle vom Schmelzpunkt 198 bis 200°C erhalten.

Beispiel 21: Tabletten enthaltend 25 mg Wirkstoff, z.B. 3-Methyl-6-pyrrolidin-1-yl-4,5-tetramethylen-benzofuran-2(3H)-on oder ein Salz, z.B. das Hydrochlorid, davon, können folgendermassen hergestellt werden:

Bestandteile (für 1000 Tabletten):

| | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzen werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 22: Kautabletten enthaltend 30 mg Wirkstoff, z.B. 5-Chlor-3-methyl-6-morpholino-2(3H)-benzofuranon oder ein Salz, z.B. das Hydrochlorid davon, können z.B. folgendermassen hergestellt werden:

Zusammensetzung (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5%ige Gelatinelösung | q.s. |

Herstellung: Alle festen Ingredienzen werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 10 mm Durchmesser mit Bruchrille auf der Oberseite verpresst.

Beispiel 23: Tabletten enthaltend 100 mg Wirkstoff, z.B. 2-(5-Chlor-2-hydroxy-4-morpholinophenyl)-propionsäure oder ein Salz, z.B. das Hydrochlorid, davon, können folgendermassen hergestellt werden:

Zusammensetzung (für 1000 Tabletten):

| | |
|---|---|
| Wirkstoff | 100,0 g |
| Lactose | 248,5 g |
| Maisstärke | 17,5 g |
| Polyäthylenglykol 6000 | 5,0 g |
| Talkum | 15,0 g |
| Magnesiumstearat | 4,0 g |
| entmineralisiertes Wasser | q.s. |

Herstellung: Die festen Ingredienzen werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

Patentansprüche

1. Verfahren zur Herstellung von Verbindungen der Formel I

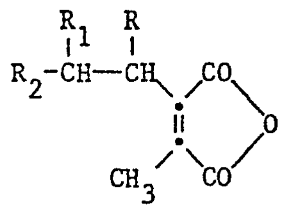

(I),

worin

R Wasserstoff, $C_{1-7}$-Alkyl, unsubstituiertes oder durch Halogen, Methoxy oder $C_{1-4}$-Alkyl substituiertes Phenyl,

$R_1$ Wasserstoff, $C_{1-7}$-Alkyl, Cyclopentyl, Cyclohexyl, unsubstituiertes oder durch Halogen, Methoxy oder $C_{1-4}$-Alkyl substituiertes Phenyl oder R und $R_1$ zusammen $-(CH_2)_3-$ oder $-(CH_2)_4-$ und

$R_2$ $-CO-C_{1-8}$-Alkyl, -COPhenyl, das durch $C_{1-4}$-Alkyl substituiert sein kann, -CN, $-COO-C_{1-4}$-Alkyl, -COO-Allyl oder -COOH bedeuten oder worin $R_1$ Wasserstoff ist und R und $R_2$ zusammen $-(CH_2)_3-CO-$ darstellen, wobei die Carbonylgruppe in dem so gebildeten Ring in m-Stellung zur Gruppe -CH- steht, indem man

a) bei R = H eine Verbindung der Formel II

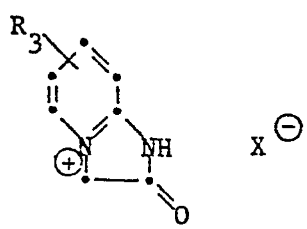

(II)

zuerst in Gegenwart einer Base mit Fumarsäure, Maleinsäure oder Maleinsäureanhydrid versetzt und anschliessend in Gegenwart einer starken Säure zu einer Verbindung der Formel III

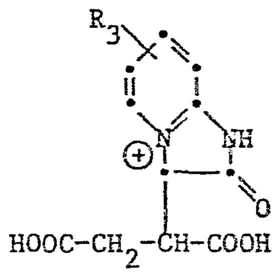 $X^{\ominus}$ (III)

umsetzt, die Verbindung der Formel III mit einer Verbindung der Formel IVa

$$CH_2=C(R_1)(R_2) \qquad (IVa)$$

zu einer Verbindung der Formel V

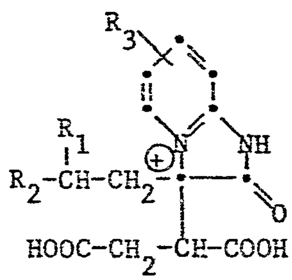 $X^{\ominus}$ (V)

umsetzt, die Verbindung der Formel V durch Erhitzen auf Temperaturen zwischen 80 und 160°C in ein Gemisch aus einer Verbindung der Formel I mit R = H und einer Verbindung der Formel VIa

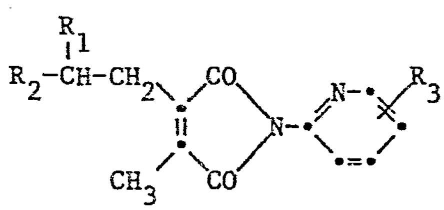 (VIa)

überführt und die Verbindung der Formel VIa mit wässriger Säure zu einer Verbindung der Formel I mit R = H hydrolysiert, oder

b) bei R ungleich H eine Verbindung der Formel II zuerst in Gegenwart einer Base mit einer Verbindung der Formel IVb

$$R'-CH=C(R_1)(R_2) \qquad (IVb)$$

versetzt und anschliessend bei Temperaturen zwischen 80 und 160°C mit Fumarsäure, Maleinsäure oder Maleinsäureanhydrid zu einem Gemisch aus einer Verbindung der Formel I mit R ungleich H und einer Verbindung der Formel VIb

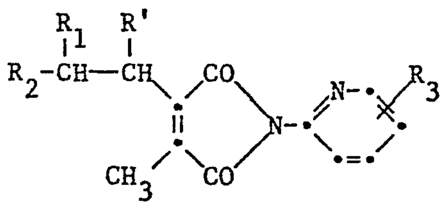

(VIb)

umsetzt und die Verbindung der Formel VIb mit wässriger Säure zu einer Verbindung der Formel I mit R ungleich H hydrolysiert, wobei R, $R_1$ und $R_2$ die unter Formel I angegebene Bedeutung haben, R' dieselbe Bedeutung wie R hat, aber ungleich H ist oder bei $R_1$ = H zusammen mit $R_2$ $-(CH_2)_3-CO-$ darstellt, wobei die Carbonylgruppe in dem so gebildeten Ring in m-Stellung zur Gruppe -CH- steht, $R_3$ Wasserstoff oder Methyl und $X^{\ominus}$ das Anion einer organischen oder anorganischen Säure bedeuten.

2. Verfahren nach Anspruch 1, dadurch gekennzeichnet, dass man bei der Verfahrensvariante a) Maleinsäure und bei der Verfahrensvariante b) Maleinsäureanhydrid einsetzt.

3. Verfahren nach Anspruch 1, dadurch gekennzeichnet, dass man als Base ein Alkalimetallhydroxid verwendet.

4. Verfahren nach Anspruch 1, dadurch gekennzeichnet, dass man die Umsetzung der Verbindung der Formel II mit Fumarsäure, Maleinsäure oder Maleinsäureanhydrid in wässrigem Medium und die Umsetzungen mit den Verbindungen der Formel IVa oder IVb in wässrig-alkoholischem Medium durchführt.

5. Verfahren nach Anspruch 1, dadurch gekennzeichnet, dass man als starke Säure Salpetersäure, Schwefelsäure oder Halogenwasserstoffsäuren, besonders HCl oder HBr, verwendet.

6. Verfahren nach Anspruch 1, dadurch gekennzeichnet, dass man die Verbindungen der Formel V oder das nach dem Zusatz von Fumarsäure, Maleinsäure oder Maleinsäureanhydrid erhaltene Reaktionsgemisch gemäss Verfahrensvariante b) in Gegenwart von Eisessig auf Temperaturen zwischen 90 und 110°C erhitzt.

7. Verfahren nach Anspruch 1, dadurch gekennzeichnet, dass man die Hydrolyse in wässrig-anorganischer Säure im Gemisch mit einem mit Wasser mischbaren organischen Lösungsmittel vornimmt.

8. Verbindungen der Formel I'

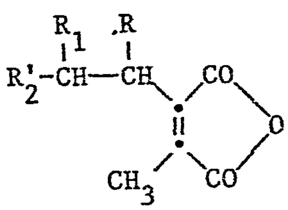

(I'),

worin

R Wasserstoff, $C_{1-7}$-Alkyl, unsubstituiertes oder durch Halogen, Methoxy oder $C_{1-4}$-Alkyl substituiertes Phenyl,

$R_1$ Wasserstoff, $C_{1-7}$-Alkyl, Cyclopentyl, Cyclohexyl, unsubstituiertes oder durch Halogen, Methoxy oder $C_{1-4}$-Alkyl substituiertes Phenyl oder R und $R_1$ zusammen $-(CH_2)_3-$ oder $-(CH_2)_4-$ und

$R_2'$ $-CO-C_{1-8}$-Alkyl, -COPhenyl, das durch $C_{1-4}$-Alkyl substituiert sein kann, -CN, $-COO-C_{1-4}$-Alkyl, -COO-Allyl oder -COOH darstellen, $R_2'$ aber, wenn R und $R_1$ Wasserstoff sind, ungleich -COOH ist, oder worin $R_1$ Wasserstoff ist und R und $R_2'$ zusammen $-(CH_2)_3-CO-$ darstellen, wobei die Carbonylgruppe in dem so gebildeten Ring in m-Stellung zur Gruppe -CH- steht.

9. Verbindungen der Formel I' nach Anspruch 8, worin $R_1$ Wasserstoff ist und R und $R_2'$ zusammen $-(CH_2)_3-CO-$ darstellen.

10. Verbindungen der Formel I' nach Anspruch 8, worin R Wasserstoff, $C_{1-7}$-Alkyl oder Phenyl und $R_1$ Wasserstoff, $C_{1-7}$-Alkyl, Cyclopentyl, Cyclohexyl oder Phenyl darstellen, wobei bevorzugt mindestens eines von R und $R_1$ Wasserstoff ist, oder R und $R_1$ zusammen $-(CH_2)_3-$ oder $-(CH_2)_4-$ bedeuten und $R_2'$ $-CO-C_{1-8}$-Alkyl, -COPhenyl, -CN, $-COOCH_3$, $-COOC_2H_5$ oder, wenn eines von R und $R_1$ ungleich Wasserstoff ist, -COOH darstellt.

11. Verbindungen der Formel I' nach Anspruch 8, worin R und $R_1$ unabhängig voneinander Wasserstoff oder $C_{1-7}$-Alkyl, besonders $C_{1-4}$-Alkyl, oder zusammen $-(CH_2)_3-$ oder $-(CH_2)_4-$ und $R_2'$ $-CO-C_{1-8}$-Alkyl, besonders $-CO-C_{1-5}$-Alkyl, bedeuten.

12. Verbindungen der Formel I' nach Anspruch 8, worin eines von R und $R_1$ Wasserstoff und das andere Wasserstoff oder $C_{1-4}$-Alkyl, besonders Methyl, und $R_2'$ $-CO-C_{1-5}$-Alkyl, besonders $-COCH_3$ oder $-COC_2H_5$, darstellen.

13. Verbindungen der Formel I' nach Anspruch 8, worin R Wasserstoff und $R_1$ $C_{1-4}$-Alkyl, besonders Methyl, und $R_2'$ $-COCH_3$ bedeuten.

14. Verbindungen der Formel VIIIa oder VIIIb

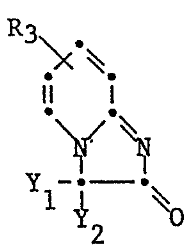

(VIIIa)

oder

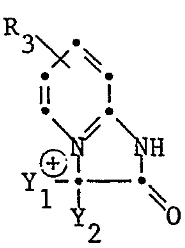

$X^{\ominus}$

(VIIIb),

worin

$Y_1$ Wasserstoff oder eine Gruppe $-CH(R)-CH(R_1)(R_2)$ und

$Y_2$ $HOOC-CH_2-CH-COOH$ darstellen oder worin

$Y_1$ eine Gruppe $-CH(R)-CH(R_1)(R_2)$ und $Y_2$ Wasserstoff bedeuten,

R Wasserstoff, $C_{1-7}$-Alkyl, unsubstituiertes oder durch Halogen,

Methoxy oder $C_{1-4}$-Alkyl substituiertes Phenyl,
$R_1$ Wasserstoff, $C_{1-7}$-Alkyl, Cyclopentyl, Cyclohexyl, unsubstituiertes oder durch Halogen, Methoxy oder $C_{1-4}$-Alkyl substituiertes Phenyl oder R und $R_1$ zusammen $-(CH_2)_3-$ oder $-(CH_2)_4-$ und
$R_2$ $-CO-C_{1-8}$-Alkyl, -COPhenyl, das durch $C_{1-4}$-Alkyl substituiert sein kann, -CN, $-COO-C_{1-4}$-Alkyl, -COO-Allyl oder -COOH bedeuten oder $R_1$ Wasserstoff ist und R und $R_2$ zusammen $-(CH_2)_3-CO-$ darstellen, wobei die Carbonylgruppe in dem so gebildeten Ring in m-Stellung zur Gruppe -CH- steht, $R_3$ Wasserstoff oder Methyl und
$X^{\ominus}$ das Anion einer organischen oder anorganischen Säure bedeuten.

15. Verbindungen der Formel IX

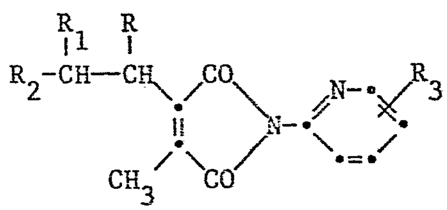

(IX),

worin
R Wasserstoff, $C_{1-7}$-Alkyl, unsubstituiertes oder durch Halogen, Methoxy oder $C_{1-4}$-Alkyl substituiertes Phenyl,
$R_1$ Wasserstoff, $C_{1-7}$-Alkyl, Cyclopentyl, Cyclohexyl, unsubstituiertes oder durch Halogen, Methoxy oder $C_{1-4}$-Alkyl substituiertes Phenyl oder R und $R_1$ zusammen $-(CH_2)_3-$ oder $-(CH_2)_4-$ und
$R_2$ $-CO-C_{1-8}$-Alkyl, -COPhenyl, das durch $C_{1-4}$-Alkyl substituiert sein kann, -CN, $-COO-C_{1-4}$-Alkyl, -COO-Allyl oder -COOH bedeuten oder $R_1$ Wasserstoff ist und R und $R_2$ zusammen $-(CH_2)_3-CO-$ darstellen, wobei die Carbonylgruppe in dem so gebildeten Ring in m-Stellung zur Gruppe -CH- steht, und $R_3$ Wasserstoff oder Methyl darstellt.

16. Verbindungen der Formeln VIIIa, VIIIb oder IX nach Anspruch 14 oder 15, worin R Wasserstoff, $C_{1-7}$-Alkyl oder Phenyl und $R_1$ Wasserstoff, $C_{1-7}$-Alkyl, Cyclopentyl, Cyclohexyl oder Phenyl darstellen, wobei bevorzugt mindestens eines von R und $R_1$ Wasserstoff ist, oder R und $R_1$ zusammen $-(CH_2)_3-$ oder $-(CH_2)_4-$ bedeuten und $R_2$ $-CO-C_{1-8}$-Alkyl, -COPhenyl, -CN, $-COOCH_3$, $-COOC_2H_5$ oder, wenn eines von R und $R_1$ ungleich Wasserstoff ist, -COOH darstelllt.

17. Verbindungen der Formeln VIIIa, VIIIb oder IX nach Anspruch 14 oder 15, worin $R_3$ Wasserstoff, R und $R_1$ unabhängig voneinander Wasserstoff oder $C_{1-7}$-Alkyl, besonders $C_{1-4}$-Alkyl, oder zusammen $-(CH_2)_3-$ oder $-(CH_2)_4-$ und $R_2$ $-CO-C_{1-8}$-Alkyl, besonders $-CO-C_{1-5}$-Alkyl, bedeuten.

18. Verbindungen der Formeln VIIIa, VIIIb oder IX nach Anspruch 14 oder 15, worin $R_3$ Wasserstoff, eines von R und $R_1$ Wasserstoff und das andere Wasserstoff oder $C_{1-4}$-Alkyl, besonders Methyl, und $R_2$ $-CO-C_{1-5}$-Alkyl, besonders $-COCH_3$ oder $-COC_2H_5$, darstellen.

19. Verbindungen der Formeln VIIIa, VIIIb oder IX nach Anspruch 14 oder 15, worin R und $R_3$ Wasserstoff und $R_1$ $C_{1-4}$-Alkyl, besonders Methyl, und $R_2$ $-COCH_3$ bedeuten.